# FASCICULE DE BREVET EUROPEEN

(11) **EP 1 678 300 B1**
(45) Date de publication et mention de la délivrance du brevet: **23.12.2009**
(21) Numéro de dépôt: 04805284.9
(22) Date de dépôt: 22.10.2004
(51) Int. Cl.: C12N 15/10, C07H 21/02, C07H 21/04

(54) **METHODE DE PRODUCTION D'ADNc PLEINE LONGUEUR PAR LIGATURATION D'ADAPTATEUR A EXTREMITE COHESIVE**
VERFAHREN ZUR HERSTELLUNG EINER VOLLÄNGEN-CDNA DURCH LIGATION EINES ADAPTERS MIT EINEM KOHÄSIVEN ENDE
METHOD FOR THE PRODUCTION OF FULL-LENGTH cDNA BY LIGATION OF AN ADAPTER WITH A COHESIVE EXTREMITY

(30) Priorité: 24.10.2003 FR 0312458
(43) Date de publication de la demande: 12.07.2006
(73) Titulaire: CENTRE NATIONAL DE LA RECHERCHE SCIENTIFIQUE (CNRS), 75016 Paris (FR); INSTITUT NATIONAL DE LA RECHERCHE AGRONOMIQUE, 75007 Paris (FR)
(72) Inventeur: CLEPET, Christian, Yves, F-92160 Antony (FR)
(74) Mandataire: Warcoin, Jacques
(86) Numéro de dépôt international: PCT/FR2004/002722
(87) Numéro de publication internationale: WO 2005/045031

(56) Documents cités:
- EP-A- 0 625 572
- CLEPET C ET AL.: "Improved full-length cDNA production based on RNA tagging by T4 DNA ligase" NUCLEIC ACIDS RESEARCH, vol. 32, no. 1001, janvier 2004 (2004-01), pages e6.1-e6.6, XP002271141
- SHIBATA Y ET AL: "Cloning full-length, Cap-Trapper-selected cDNAs by using the single-strand linker ligation method" BIOTECHNIQUES, EATON PUBLISHING, NATICK, US, vol. 30, no. 6, juin 2001 (2001-06), pages 1250-1254, XP002197302 ISSN: 0736-6205
- CHENCHIK A ET AL: "FULL-LENGTH CDNA CLONING AND DETERMINATION OF MRNA 5' AND 3' ENDS BY AMPLIFICATION OF ADAPTOR-LIGATED CDNA" BIOTECHNIQUES, EATON PUBLISHING, NATICK, US, vol. 21, no. 3, 1 septembre 1996 (1996-09-01), pages 526-534, XP000635197 ISSN: 0736-6205
- MARUYAMA K ET AL: "OLIGO-CAPPING: A SIMPLE METHOD TO REPLACE THE STRUCTURE OF EUKARYOTIC MRNAS WITH OLIGORIBONUCLEOTIDES" GENE, ELSEVIER BIOMEDICAL PRESS. AMSTERDAM, NL, vol. 138, 1994, pages 171-174, XP001008855 ISSN: 0378-1119

## Description

La présente invention a pour objet une méthode de production d'ADNc pleine longueur par ligaturation d'un oligonucléotide à l'extrémité 5' d'un ARN grâce un adaptateur double brin à extrémité cohésive et une ligase.

### ART ANTERIEUR

En biologie moléculaire, l'étude des ADNc pleine longueur demeure une approche incontournable pour déterminer la structure des gènes. L'établissement de librairies d'ADNc de qualité constitue un préliminaire indispensable dans différents programmes de recherche en génomique. La connaissance de la séquence complète des transcrits permet par exemple de choisir des sondes de spécificité optimale pour la constitution de puces à ADN, ou dans des projets d'analyse fonctionnelle par « gene silencing ». Les construits d'ADNc portant la totalité du cadre de lecture permettent par exemple d'étudier la structure de la protéine, sa localisation cellulaire, ses partenaires éventuels, etc.
Un ADNc est dit de pleine longueur lorsqu'il couvre la totalité de la séquence d'un transcrit. Cette terminologie est communément utilisée dans le cas des ARN messagers (ARNm) eucaryotes pour lesquels l'ADNc pleine longueur contiendra notamment la région directement en aval de la coiffe en 5'.

L'isolement des ADNc pleine longueur est une problématique difficile qui a inspiré un grand nombre de travaux. La méthode idéale de production d'ADNc pleine longueur doit être la plus spécifique possible de l'extrémité 5' coiffée des ARN messager afin de pouvoir contre-sélectionner les ADNc tronqués. Dans la pratique, ce gain de spécificité se fait généralement au détriment de l'efficacité globale du protocole, ce qui peut freiner considérablement le clonage des ARN messagers les plus rares ou instables, ou les plus difficiles à convertir en ADN complémentaires.

Dans la méthode de production d'ADNc pleine longueur de Sekine et col. (brevet EP 0 625 572), après traitement de la préparation d'ARN par la phosphatase alcaline, les ARNm sont décoiffés par la pyrophosphatase acide du tabac et ligaturés à un oligonucléotide par la T4 RNA ligase. Les ARN obtenus sont ensuite convertis en ADNc par une transcriptase inverse.
Dans ce protocole, la production d'ADNc pleine longueur est limitée au niveau de l'étape de fixation de l'adaptateur sur l'ARN ; il est bien connu en effet par l'homme du métier que le raboutage et la ligaturation de deux fragments d'acide nucléique monocaténaires présentent une efficacité réduite. En outre ce procédé d'étiquetage présente des problèmes de spécificité du fait que n'importe quelle extrémité 3'OH présente dans le milieu réactionnel peut servir de substrat de ligaturation à la place de l'adaptateur et conduire à la formation de chimères ou autre concatémères et aboutir de ce fait à l'inactivation et la perte des ARNm les plus difficiles à cloner.

### DESCRIPTION DE L'INVENTION

De manière surprenante et inattendue, les inventeurs ont montré que la méthode de production d'ADNc pleine longueur de la présente invention permet de résoudre ces problèmes par l'utilisation d'un adaptateur double brin à extrémité cohésive créant une structure double brin au niveau de la jonction avec l'ARN et d'une ligase pour la ligaturation d'un oligonucléotide à l'extrémité 5' des ARN. Les inventeurs ont montré que ladite méthode permet d'augmenter l'efficacité de la ligaturation. L'utilisation d'adaptateurs double brin à extrémité cohésive apporte des améliorations significatives pour le positionnement de l'oligonucléotide au niveau de l'extrémité 5' de l'ARN. La structure double brin générée au niveau de la jonction avec l'ARN permet en outre de catalyser la ligaturation au moyen de la T4 DNA ligase dont la constante d'affinité (10-8 M<Km<10-7 M pour des polynucléotides au sein de structures double brin) est significativement plus faible que pour la T4 RNA ligase (Km>1mM pour des polynucléotides)
Outre un gain d'efficacité, cette méthode présente une spécificité accrue par rapport au protocole de Sekine et col. (brevet EP 0 625 572). Dans ce protocole tout polynucléotide présentant une extrémité 3'OH constitue un site accepteur potentiel pour la T4 RNA ligase et peut donc être fixé sur l'extrémité 5' phosphate des ARNm. Outre les problèmes de bruit de fond liés à la présence des concatémères et autres chimères qui peuvent être ainsi générés, ce type d'événement peut conduire à l'inactivation et à la perte des transcrits les plus rares. La spécificité de ligaturation conférée par la T4 DNA ligase qui exerce son activité sur des « nicks » au sein de structures bicaténaires, permet d'éviter ce type de bruit de fond.
On signalera enfin que l'activité de la T4 RNA ligase étant plus élevée sur certaines séquences que d'autres, cela pourrait conduire à une représentation inégale des transcrits dans les banques d'ADNc. Un tel biais de séquence n'a jamais été décrit pour la T4 DNA ligase.

L'objet de la présente invention est donc une méthode de production d'ADNc pleine longueur comprenant une étape a) de déphosphorylation des fragments d'acides nucléiques non pourvus d'une coiffe en 5', une étape b) de suppression de la coiffe de l'extrémité 5' des ARN coiffés, une étape c) de ligaturation d'oligonucléotide à l'extrémité 5' d'ARN obtenus à l'étape b) et une étape d) de synthèse des ADNc pleine longueur, par rétro-transcription des ARN obtenus à l'étape c), par une transcriptase inverse, ladite méthode étant caractérisée en ce que l'étape c) de ligaturation d'oligonucléotide est réalisée par une ligase et un adaptateur double brin à extrémité cohésive, ledit adaptateur double brin à extrémité cohésive comprenant un brin supérieur portant un site 3'OH accepteur de ligase pour le couplage avec le 5' des ARN et un brin inférieur présentant en 5' une extension oligonucléotidique monocaténaire de séquence aléatoire pouvant s'apparier avec l'extrémité 5' des ARN.
L'étape a) est réalisée de manière conventionnelle, connue par l'homme du métier et peut entre autre être réalisée par la phosphatase alcaline (Maniatis et Al, cf. techniques générales de biologie moléculaire)
L'étape b) est réalisée de manière conventionnelle, connue par l'homme du métier et peut entre autre être réalisée par la pyrophosphatase acide du tabac.
L'étape d) est réalisée de manière conventionnelle, connue par l'homme du métier et peut entre autre être réalisée par l'appariement d'une amorce à l'extrémité 3' d'un ARN obtenu à l'étape c), l'élongation 5'-3' de l'amorce par la transcriptase inverse pour former le premier brin d'ADNc, la dégradation du brin d'ARN par un
traitement alcalin, et la synthèse du deuxième brin d'ADNc par une ADN polymérase ADN dépendante. (Maniatis et Al, cf. techniques générales de biologie moléculaire)

Par ARN coiffé, on entend désigner de manière préférentielle selon la présente invention des ARN messagers.

Par ligase on entend désigner selon la présente invention une DNA ligase, une RNA ligase ou une combinaison de RNA ligase et de DNA ligase

De manière préférentielle, la ligase utilisée selon la présente invention est une DNA ligase, et plus particulièrement la T4 DNA ligase

Par adaptateur double brin à extrémité cohésive, on entend désigner selon la présente invention une molécule d'acide nucléique double brin comprenant un brin supérieur portant un site 3'OH accepteur de ligase pour le couplage avec le 5' des ARN et un brin inférieur présentant en 5' une extension oligonucléotidique monocaténaire de séquence aléatoire pouvant s'apparier avec l'extrémité 5' des ARN.

De manière optionnelle, selon la présente invention, le brin inférieur de l'adaptateur double brin à extrémité cohésive peut être bloqué par un groupement amine en 3'.

Par adaptateur double brin à extrémité cohésive constitué d'une molécule d'acide nucléique on entend désigner selon la présente invention un adaptateur double brin à extrémité cohésive constitué d'ADN ou d'ARN ou d'une composition mixte ADN/ARN.

De manière préférentielle, le brin supérieur de l'adaptateur double brin est composé de 24 désoxyribonucléotides en 5' suivi d'un fragment de 8 ribonucléotides en 3'.
De manière préférentielle, le brin inférieur de l'adaptateur double brin comporte en 5' une extension oligonucléotidique monocaténaire de séquence aléatoire de 6 bases. Il est à noter que ladite extension oligonucléotidique monocaténaire de séquence aléatoire peut être constituée de n'importe quel type de base pouvant s'apparier avec l'ARN et peut comporter moins de 6 bases, ou plus de 6 bases, sans que cela ne modifie la présente invention.

L'utilisation de ce type d'adaptateurs double brin à extrémité cohésive et de la T4 DNA ligase peut trouver une application chaque fois qu'un adaptateur oligonucléotidique doit être ligaturé sur des ARN.
En particulier cette méthode est utile pour ligaturer un adaptateur sur l'extrémité 5' phosphate d'un ARN de séquence non connue, en utilisant des adaptateurs double brin à extrémité cohésive du même type que ceux décrits précédemment.
Si nécessaire un phosphate pourra être rajouté au préalable sur l'extrémité 5' des ARN, au moyen de l'activité polynucléotide kinase du bactériophage T4, afin de créer un site donneur pour la T4 DNA ligase, pouvant réagir avec le site accepteur 3' hydroxyle de l'adaptateur.
Cette stratégie d'étiquetage peut constituer un procédé alternatif permettant des gains d'efficacité dans les expériences d'immunoprécipitation de ribonucleoproteines (RIP).

La présente invention a également pour objet une méthode de ligaturation d'un adaptateur double brin à extrémité cohésive à l'extrémité 5' d'un ARN, comprenant les étapes :
a) Obtenir un ARN simple brin présentant une extrémité 5'phosphate
b) Préparer les deux brins d'un adaptateur à extrémité cohésive comprenant un brin supérieur portant un site 3'OH accepteur de ligase pour le couplage avec le 5' des ARN et un brin inférieur à extrémité cohésive présentant en 5' une extension oligonucléotidique monocaténaire de séquence aléatoire pouvant s'apparier avec l'extrémité 5' phosphate des ARN
c) Mélanger ledit brin supérieur avec ledit brin inférieur de façon à former un adaptateur double brin
d) Mélanger ledit adaptateur avec ledit ARN simple brin pour permettre à l'extrémité 5' dudit brin inférieur de s'hybrider avec ledit simple brin d'ARN
e) Ligaturer ledit adaptateur avec ledit simple brin d'ARN par une ligase.

Par ARN, on entend désigner selon la présente invention des ARN coiffés ou des ARN non coiffés.
Si par ARN on entend des ARN coiffés, alors l'étape a) est une suppression de la coiffe de l'extrémité 5' des ARN coiffés. Ladite suppression de la coiffe peut être réalisée par exemple par la pyrophosphatase acide du tabac. De manière préférentielle les ARN coiffés sont des ARN messagers.
Si par ARN, on entend des ARN non coiffés et pourvus d'une extrémité 5'OH, alors l'étape a) est une phosphorylation de l'extrémité 5'OH des ARN non coiffés. Ladite phosphorylation peut être réalisée par exemple par la T4 polynucléotide kinase.
Si par ARN, on entend des ARN non coiffés et pourvus d'une extrémité 5'phosphate, alors l'étape a) consiste à isoler lesdits ARN.
De manière préférentielle, la ligase utilisée à l'étape e) est une DNA ligase, une RNA ligase ou une combinaison de RNA ligase et de DNA ligase. De manière plus préférentielle, la ligase utilisée est une DNA ligase, et plus particulièrement la T4 DNA ligase.

De manière optionnelle, le brin inférieur de l'adaptateur double brin à extrémité cohésive peut être bloqué par un groupement amine en 3'

Par adaptateur double brin à extrémité cohésive, on entend désigner selon la présente invention un adaptateur double brin à extrémité cohésive constitué d'ADN ou d'ARN ou d'une composition mixte ADN/ARN.

De manière préférentielle, le brin supérieur de l'adaptateur double brin est composé de 24 désoxyribonucléotides en 5' suivi d'un fragment de 8 ribonucléotides en 3'.

De manière préférentielle, le brin inférieur de l'adaptateur double brin comporte en 5' une extension oligonucléotidique monocaténaire de séquence aléatoire de 6 bases.

Il est à noter que ladite extension oligonucléotidique monocaténaire de séquence aléatoire peut être constituée de n'importe quel type de base pouvant s'apparier avec l'ARN et peut comporter moins de 6 bases, ou plus de 6 bases, sans que cela ne modifie la présente invention.

La présente invention a également pour objet un Kit pour la production d'ADNc pleine longueur caractérisé en ce qu'il comprend au moins :
o phosphatase alcaline et son tampon réactionnel
o pyrophosphatase acide du tabac et son tampon réactionnel
o une ligase, ou un cocktail de ligases, comprenant en particulier la T4 DNA ligase et son tampon réactionnel
o un adaptateur double brin à extrémité cohésive, ledit adaptateur double brin à extrémité cohésive comprenant un brin supérieur portant un site 3'OH accepteur de ligase pour le couplage avec le 5' des ARN et un brin inférieur présentant en 5' une extension oligonucléotidique monocaténaire de séquence aléatoire pouvant s'apparier avec l'extrémité 5' des ARN.
o une transcriptase inverse
o une ADN polymérase ADN dépendante
o des amorces oligonucléotidiques pour la polymérisation de l'ADN

### LEGENDE DES FIGURES

**Figure 1**
   Stratégie de synthèse d'ADNc pleine longueur
**Figure 2**
   Analyse des produits de RACE PCR sur gel à 2% d'agarose coloré au bromure d'éthydium. 12 µl de produit PCR (1 - 6) amplifié avec les amorces P1 et rbc402.L (1 - 3) ; P1 (4) ; rbc402.L (5) ; rbc64.U et rbc402.L (6) ; sur ADNc (3 - 6) ; sur ADN génomique (1) ; témoin sans ADN (2). L : 100 bp ladder (Life Technologies).
**Figure 3**
   Electrophérogramme de la séquence d'un clone de la RACE PCR basée sur l'amorce rbc402.L. la flèche vers la droite indique la séquence (complémentaire inversée) correspondant à l'extrémité 5' du transcrit ats1A, fusionnée avec la séquence de l'adaptateur P1 (flèche vers la gauche).
**Figure 4**
   Analyse des clones de RACE PCR. La séquence de la région adjacente à l'adaptateur est présentée pour 10 clones. Les fragments d'ADNc obtenus correspondent à 3 gènes. Le numéro d'accession et la séquence génomique encadrant le site (+1) d'initiation de transcription décrits dans Genbank sont présentés pour chaque gène. N : nombre de clones. Cap site : position relative de l'extrémité 5' des fragments clonés par rapport au +1 annoté dans Genbank.

### EXEMPLE

### 1) Matériel et méthodes

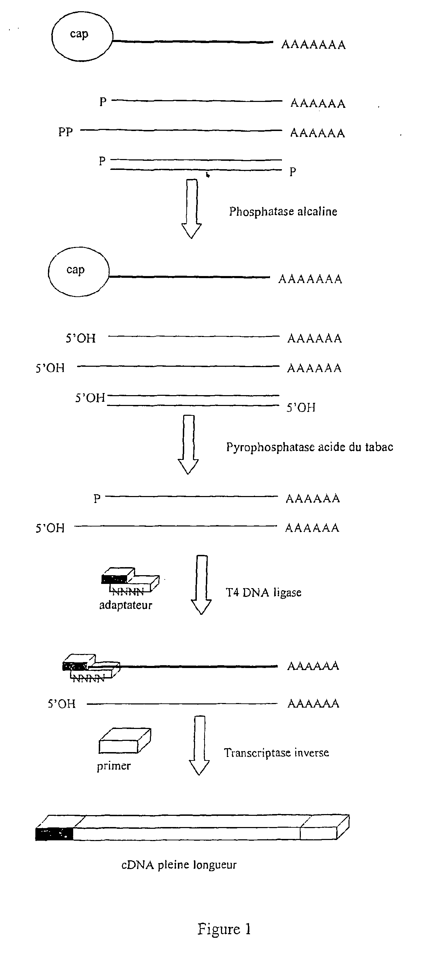

La méthode de production d'ADNc selon la présente invention est présentée en figure 1.

### - Les oligonucléotides

Les séquences sont orientées de 5' vers 3'. N correspond à n'importe quel type de base pouvant s'apparier avec l'ARN ; V correspond à l'un des 3 desoxyribonucléotides dA, dG ou dC; rC, rT,rG sont des ribonucléotides ; a indique un groupement NH2 sur le carbone 3'.

**Tableau 1 : oligonucléotides**

| | |
|---|---|
| P1 (SEQ ID n°1) | GGGGACAAGTTTGTACAAAAAAGCtArGrGrCrTrGrGrG |
| P2 (SEQ ID n°2) | NNNNNNCCCAGCCTGCa |
| P3 (SEQ ID n°3) | GGGGACCACTTTGTACAAGAAAGCTGGGTTTTTTTTTTTTTTTTTTTTVN |
| Rbc64.U (SEQ ID n°4) | AATGGCTTCCTCTATGCTCTC |
| Rbc402.L (SEQ ID n°5) | TGCGGAGAAGGTAGTCAACTTCCT |
| M13fwd (SEQ ID n°6) | TGTAAAACGACGGCCAGTGA |
| M13rev (SEQ ID n°7) | GGAAACAGCTATGACCATGAT |
| Univ221.U (SEQ ID n°8) | TGTAAAACGACGGCCAGTCTTA |
| Univ221.L (SEQ ID n°9) | CAGGAAACAGCTATGACCATGT |
| P1per (SEQ ID n°10) | GGGGACAAGTITGTACAAAAAAGCAGGCT |
| P3pcr (SEQ ID n°11) | GGGGACCACTTTGTACAAGAAAGCTGGGT |

### - Préparation de l'adaptateur

L'adaptateur a été préparé en mélangeant dans un microtube 40 µM de chacun des oligonucléotides P1 et P2, dans un tampon contenant 10 mM de NaCl et 10 mM de Tris-HCl (pH 7,5). Le microtube a été chauffé à 70 °C dans un bécher contenant 100 ml d'eau et laissé revenir progressivement à température ambiante pendant environ 20 mn. La réaction d'hybridation a été aliquotée et conservée à-80 °C.

### - Préparation des ARN

Les ARN totaux ont été purifiés par la méthode au Trizol (Invitrogen) à partir de tissus aériens végétatifs d'Arabidopsis thaliana, en suivant les conditions recommandées par le fabricant.

### - Fixation de l'adaptateur sur le site de la coiffe des ARN messagers

### Déphosphorylation:

2 µg d'ARN total ont été digérés par 2 unités de phosphatase alcaline (Roche) dans 20 µl de tampon contenant 50 mM de Tris-HCl (pH 8,5), 5 mM de MgCl2, et 40 unités d'inhibiteur de ribonucléase (RNasin, Promega) pendant 1h à 37 °C. Le mélange réactionnel a été chauffé à 65 °C 10 mn, complété à 100 µl avec H2O extrait au phénol:chloroforme et précipité en présence de 300 mM d'acétate de sodium, 20 µg de glycogène, et 2 volumes d'éthanol absolu, pendant 20 mn à -20°C. L'ARN a été récupéré par centrifugation à 14000 g pendant 20 min, lavé par 200 µl d'éthanol à 75% et séché en laissant le tube ouvert sur la paillasse pendant 5 mn.

### Suppression de la coiffe des ARN messager:

L'ARN a ensuite été digéré pendant 1h à 37°C, par 2 unités de la pyrophosphatase acide du tabac (Epicentre) dans 20 µl du tampon recommandé par le fabricant, supplémenté par 40 unités de RNasin. Le volume réactionnel a été complété à 100 µl avec H2O, extrait au phénol-chloroforme et précipité à l'éthanol comme décrit ci-dessus. Le culot d'ARN a été lavé par l'éthanol à 75% et séché 5 mn sur la paillasse.

### Ligaturation de l'oligonucléotide:

Les ARN débarrassés de la coiffe en 5' sont repris dans 6,5 µl d'H2O, chauffés à 65°C pendant 5 mn, équilibrés à 25 °C et mélangés dans un volume final de 10 µl avec 50 mM de Tris-HCl (pH 7,5), 10 mM de MgC12, 10 mM de dithiothreitol (DTT), 1 mM d'ATP, 25 µg/ml d'albumine de serum de boeuf (BSA), 5 % de polyethylene glycol 8000, 20 unités de Rnasin, 2 µM d'adaptateur double brin et 1000 unités de T4-DNA ligase à forte concentration (New England Biolabs). La réaction a été incubée pendant 3h à 25 °C. Le volume réactionnel a été complété à 100µl avec H2O, extrait au phénol-chloroforme, et précipité en présence de 300 mM d'acétate de sodium, 20 µg de glycogène et 2 volumes d'éthanol, pendant la nuit à -20°C. Le culot d'ARN a été lavé par l'éthanol à 75% et séché 5 mn sur la paillasse.

### - Transcription inverse

Le culot d'ARN a été repris dans 8 µl d'H2O, mélangé avec l'amorce oligonucléotidique P3 (50 pmoles) et les 4 dNTP (10 nmoles de chaque), dans un volume final de 12 µl et chauffé à 65°C pendant 5 mn. La solution a été équilibrée à 48°C et complétée par 40 unités de Rnasin, 10 mM de DTT, 50 mM de Tris-HCl (pH 8,3), 75 mM de KCl, 3 mM de MgCl2, et 200 unités de M MLV Superscript III (Invitrogen), dans un volume final de 20 µl. Après 50 mn à 48°C, la réaction a été arrêtée en chauffant 15 mn à 70°C. Les ADN complémentaires ont été congelés à -20 °C

### - PCR

Les PCR ont été effectuées en utilisant 1,5 unités de polymérase AmpliTaq Gold (Roche) dans 15 µl de tampon contenant 15 mMTris-HCl (pH 8,0), 50 mM KCl, 1,5 mM MgC12, 200 µM de chaque dNTP, et 0,2 µM de chaque amorce oligonucléotidique, pour les contrôles génomiques, 60 ng d'ADN d'A. thaliana ont été utilisés. Pour le criblage de la banque de RACE PCR et pour la production de matrice de séquençage, les PCR ont été effectuées sur 1 µl de chaque clone conservé à -80°C dans 4% de glycérol, avec les amorces (rbc64.U, rbc402.L) ou (M13fwd, M13rev). Pour le criblage et la production de matrice de séquençage de la banque d'ADNc pleine longueur, la paire d'amorces (univ221.U, univ221.L) a été utilisée. Sauf avis contraire les PCR ont été effectuées dans les conditions suivantes : 8 mn à 94 °C puis {94 °C 10 sec, 60 °C 10 sec, 72 °C 1 mn} pendant 30 cycles et une étape finale de 2 mn à 72°C. Les réactions ont été analysées sur gel d'agarose à 2%, coloré au bromure d'éthydium.

### - Clonage

Pour les expériences d'amplification des extrémités de fragments d'ADNc (RACE PCR), 1 µl d'ADNc ont été amplifiés avec la paire d'amorces (P1, rbc402.L) avant d'être clonés dans le vecteur type « T/A cloning » pGEM-T (Promega). Un µl du produit de RACE PCR a été incubé avec 20 ng du vecteur de clonage, pendant 1h à 20°C, dans 5 µl de tampon contenant 50 mM de Tris-HCl (pH 7,5), 10 mM de MgCl2, 10 mM de DTT, 1 mM d'ATP, 25 µg/ml de BSA et 200 unités de T4 DNA ligase (Biolabs).
Pour construire la banque d'ADNc pleine longueur, 3µl d'ADNc a été soumis à une série de 12 cycles d'amplification PCR dans les conditions suiivantes {94 °C 10 sec, 66 °C 20 sec, 72 °C 5 mn}, dans un volume final de 20 µl avec les amorces P1pcr et P2per. Les fragments PCR de taille supérieure à 1 kb ont été fractionnés sur un gel à 1,2% d'agarose Seakem (Tebu) et extraits du gel par électroélution. L'ADN élué a été précipité en présence de 300 mM d'acétate de sodium, 20 µg de glycogène et 2 volumes d'éthanol, pendant la nuit à -20°C. Le culot d'ADN a été lavé par l'éthanol à 75%, séché 5 mn sur la paillasse et repris dans 10 µl de tampon Tris-HCl pH 7,5 10 mM, EDTA 1 mM. Les ADNc ainsi préparés ont été insérés dans le vecteur pDONR221 (Invitrogen) par recombinaison in vitro, dans un volume final de 20 µl, à 24°C sur la nuit, à l'aide du kit Gateway et en suivant les recommandations du fabriquant (Invitrogen).
Après désalage des réactions sur minicolonnes de sepharose 4LB (Amersham Pharmacia) le 1/5ème des produits de ligaturation (RACE PCR) ou de recombinaison par Gateway (ADNc pleine longueur) ont été utilisés pour transformer par électroporation la souche E. coli DH10B {F- mcrA D(mrr-hsdRMS-mcrBC) F 801acZDM15 DlacX74 deoR recAl endA1 araD139 D(ara,leu)7697 galU galK 1 - rpsL (StrR) nupG tonA} commercialisée par Invitrogen, avec un électroporateur de marque Life Technologies en suivant les conditions préconisées par le constructeur. Les cellules transformées ont été incubée pendant 45 mn en milieu SOC, avant d'être étalées sur des boîtes de Petri contenant du milieu LB gélosé, supplémenté par 50 µg/ml de carbeniciline, 20 µg/ml de 5-bromo-4-chloro-3-indolyl-β-D-galactoside et d'isopropylthio-β-D-galactoside pour le clonage dans pGEM-T, ou supplémenté par 50 µg/ml de kanamycine pour le clonage dans le pDONR221.

### - Séquençage

Les fragments d'ADNc ont été isolés par PCR avec deux amorces localisées de part et d'autre du site de clonage : soit (M13fwd , M13rev) pour les construits dans pGEM-T et (Univ221.U , Univ221.L) pour les construits dans pDONR221. Les matrices ont été purifiées en utilisant le système MinElute de Qiagen et séquencées en fluorescence sur des appareils ABI.

### - Analyse de séquence

Les séquences ont été analysées par BLAST contre la base Genbank, en utilisant la version d'avril 2003 pour les produits de RACE PCR, et la version du 8 août 2003 pour les clones de la banque d'ADNc pleine longueur. Pour l'analyse des clones d'ADNc pleine longueur, le locus génomique correspondant à chaque fragment d'ADNc a été identifié en utilisant la base de données Flagdb22, et une séquence génomique de 3 kb ancrée en position 1000 sur la première base du cadre de lecture annoté par l'AGI, et couvrant 2000 bases en amont (ie vers le promoteur du gène) et 1000 bases en aval de cette base a été isolé pour chacun des gènes. Chaque séquence de 3 kb a été utlisée comme requête pour questionner par BLAST une base de données regroupant les séquences des 154 fragments clonés d'une part et une base de données constituées à partir de la totalité des séquences d'ADNc (EST, RNA, cDNA) présentes dans Genbank sous le champ Arabidopsis thaliana d'autre part.
Remarque: Afin de pouvoir éventuellement utiliser le système de clonage Gateway breveté par Invitrogen, les sites Gateway attB1 et attB2 de recombinaison in vitro ont été intégrés dans les adaptateurs P1/P2 et P3. Ce système de clonage a effectivement été utilisé pour la construction de la banque d'ADNc pleine longueur mais pas pour le clonage des produits de RACE PCR qui ont été clonés par ligaturation. On insistera sur le fait que la méthode selon la présente invention n'est dépendante ni du système de clonage Gateway ni de la présence des séquences attB1 et attB2 sur les adaptateurs utilisés. D'autres types de séquence d'adaptateur, un autre type de vecteur que le pDONR221 et un clonage « classique » par ligaturation aurait tout aussi bien pu être utilisé pour construire la banque d'ADNc pleine longueur. L'utilisation du système Gateway doit être considérée comme une variante de clonage, avec ses avantages et ses inconvénients, pouvant « agrémenter » la stratégie décrite de production d'ADNc pleine longueur.

### 2) Résultats et discussion

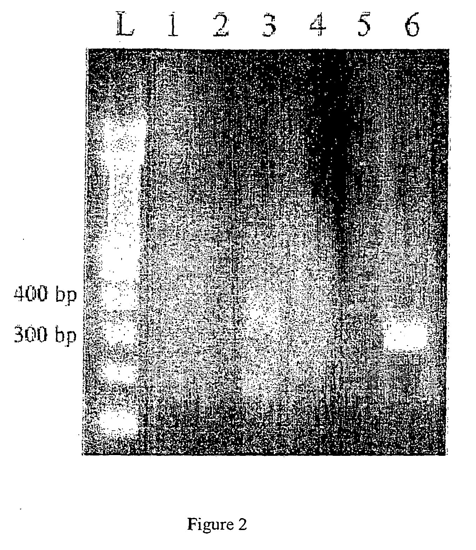

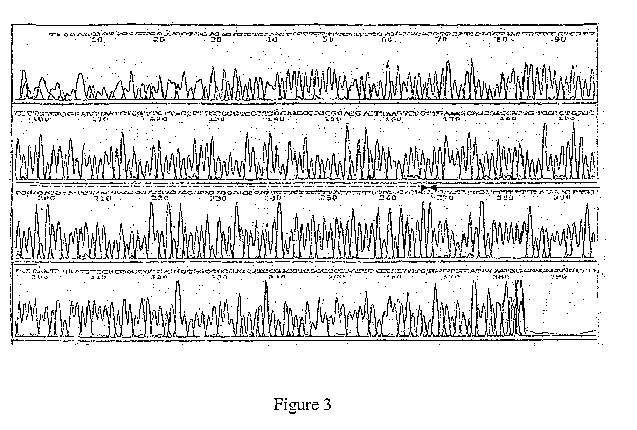

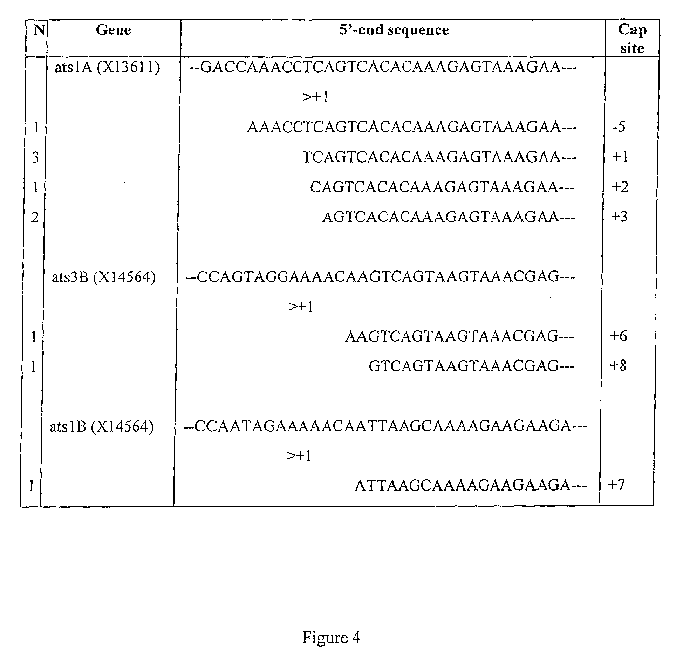

La nouvelle méthode d'étiquetage des ARN et son application pour la production d'ADNc pleine longueur a été validée de façon convergente par une expérience de RACE PCR et par la construction et l'analyse d'une banque d'ADNc. La RACE PCR a été effectuée sur un système expérimental déjà documenté : une amorce de PCR (rbc402.L, Tableau 1) a été choisie sur une séquence homologue entre quatre gènes d'Arabidopsis thaliana ; cette amorce reconnaît une cible identique à 100% sur les gènes ats1B, ats2B et ats3B, et présente une base de différence dans sa partie 5', pour le gène ats1A. L'amorce est orientée vers le site d'initiation de transcription et se trouve à environ 300 bases de celui-ci sur l'ADNc. Des ARN totaux d'Arabidopsis thaliana ont été fraîchement purifiés et couplés en 5' avec l'étiquette P1 au moyen de la T4 DNA ligase, selon le protocole énoncé ci-dessus. Des ADNc ont été syntétisés à partir des ARNm couplés à l'étiquette 5' et d'amorces P3 (Tableau 1), et amplifiés par PCR avec les amorces rbc402.L et P1. L'analyse sur gel de la réaction de RACE PCR fait apparaître une bande compatible avec le produit attendu de 350 paires de bases et absente des témoins de PCR (Figure 2). Le produit de la RACE PCR a été cloné dans le vecteur pGEM-T et les construits de 10 clones amplifiables par PCR avec les amorces (rbc64.U, rbc402.L) ont été séquencés. Ces fragments d'insertions correspondent à la région 5' des ADNc ats1A, ats1B et ats3B, fusionnés selon la configuration attendue avec la séquence de l'étiquette P1 ; à titre d'exemple, l'électrophérogramme de la séquence complémentaire inversée d'un clone portant l'extrémité 5' du transcrit ats1A est présenté en figure 3. La figure 4 résume les résultats de l'analyse des clones : en particulier 3 ADNc « ats1A » correspondent exactement au site d'initiation de transcription (SIT) décrit dans Genbank pour ce gène, et un fragment « ats1A » présente l'incorporation de 5 bases supplémentaires en direction du promoteur. Les 6 autres construits correspondent à des fragments d'ADNc ayant une extrémité 5' localisée à 1 base ou quelques bases en aval des sites d'initiation de transcription décrits dans Genbank respectivement pour ats1A, ats3B et ats1B.
L'évaluation de la stratégie a ensuite été recherchée via la construction et l'analyse d'une banque d'ADNc pleine longueur. Arabidopsis thaliana constitue un système expérimental de choix pour valider une méthode de production d'ADNc pleine longueur, grâce à l'étendue et la qualité des données accumulées chez cet organisme. On dispose en effet de la séquence génomique extensivement annotée et de plus de 220000 séquences d'ADNc (répertoriés sous les champs EST, RNA ou cDNA), dont une part importante est issue de projets de caractérisation d'ADNc pleine longueur utilisant des technologies concurrentes. Une banque d'ADNe a donc été construite à partir des ARN ligaturés à l'étiquette 5' et rétro-transcrits avec l'amorce P3. Les ADNc ont été amplifiés par PCR avec les amorces P1pcr et P3pcr (Tableau 1), fractionnés sur gel d'agarose et clonés par le système Gateway (Invitrogen) dans le vecteur pDONR221. Un total de 154 clones recombinants ont été séquencés et soumis à une série d'analyses bioinformatiques afin d'évaluer la qualité des ADNc obtenus. En l'absence d'une « séquence signature » connue qui permettrait de définir si un fragment d'ADNc est complet ou non, l'évaluation des ADNc a été établi sur les considérations suivantes : un premier critère consiste à vérifier si la totalité de la séquence codante (ORF) est ou non portée par le fragment d'ADNc ; on s'est référé pour ce faire aux annotations de l'Arabidopsis Genome Initiative (AGI) (The Arabidopsis Genome Initiative. Analysis of the genome sequence of the flowering plant Arabidopsis thaliana. Nature 408, 796-815 (2000).). En deuxième lieu, la position de l'extrémité 5' de chaque ADNc a été comparée à celles de la totalité des séquences d'ADNc relatives aux transcrits considérés et présentes dans Genbank (release du 13 aout 2003). Le résultat des analyses clone à clone est regroupé dans le tableau 2 ; un bilan est présenté dans le tableau 3.

**Tableau 2**

| **clone** | **locus ID** | **GB 5'** | **ATG** | **note** |
|---|---|---|---|---|
| a192B10 | AT5G44730 | **absent** | **-143** | (6) |
| a189D5 | AT5G19550 | **-72** | **-167** | |
| a189E2 | AT5G25752 | **-34** | **-61** | |
| a209D1 | AT2G35450 & AT2G35440 | **-19** | **-8** | (1) |
| a189E12 | AT4G17050 | **-16** | **-88** | |
| a209E4 | | **+55** | **-26** | |
| a188F11 | | **+60** | **-21** | |
| a209D10 | AT2G42240 & AT2G42245 | **-15** | **-72** | (2) |
| a209A8 | AT1G06040 | **-14** | **-244** | |
| a189F12 | AT4G01310 | **-10** | **-65** | |
| a189F9 | AT1G71190 | **-5** | **-50** | |
| a209B4 | AT1G29930 | **-5** | **-71** | |
| a189A4 | | **+1** | **-66** | |
| a189E11 | | **+1** | **-66** | |
| a189A11 | | **+1** | **-66** | |
| a188G8 | | **+1** | **-66** | |
| a192C3 | | **+1** | **-66** | |
| a192C4 | | **+1** | **-66** | |
| a209E5 | | **+1** | **-66** | |
| a189C7 | | **+1** | **-66** | |
| a189D4 | | **+1** | **-66** | |
| a192B7 | | **+2** | **-65** | |
| a188B7 | | **+5** | **-62** | |
| a063B1 | AT1G09590 | **-3** | **-15** | |
| a197bG2 | AT1G06680 | **-2** | **-63** | |
| a209C5 | | **+4** | **-58** | |
| a197bB6 | | **+4** | **-58** | |
| a189D11 | | **+41** | **-21** | |
| a209D2 | AT1G43800 | **+1** | **-51** | |
| a209A7 | AT3G17410 | **+1** | **-163** | |
| a189C1 | AT4G05320 | **+1** | **-68** | |
| a189B5 | AT5G57850 | **+1** | **-39** | |
| a197bH5 | AT5G57930 | **+1** | **-86** | |
| a197bG1 | AT1G19570 | **+1** | **-42** | |
| a192C11 | AT3G12930 | **+1** | **-56** | |
| a209D12 | AT3G44860 | **+1** | **-56** | |
| a192A11 | | **+1** | **-56** | |
| a063D1 | AT2G34430 | **+1** | **-62** | |
| a192C6 | | **+2** | **-62** | |
| a189E9 | | **+2** | **-62** | |
| a209E2 | AT5G22340 | **+1** | **-36** | |
| a209C8 | AT1G15430 | **+2** | **-136** | |
| a189D12 | AT1G78630 | **+2** | **-40** | |
| a189B10 | AT3G54890 | **+3** | **-66** | |
| a197bH1 | | +16 | **-53** | |
| a209D5 | AT1G14290 | **+3** | **-166** | |
| a209D8 | AT4G15545 | **+3** | **-10** | |
| a189C6 | AT1 G44575 | **+3** | **-43** | |
| a209A1 | | **+7** | **-39** | |
| a192A9 | AT1G20620 | **+3** | **-57** | |
| a209D6 | | **+4** | **-56** | |
| a209B9 | AT3G08940 | **+3** | **-39** | |
| a189D8 | | **+3** | **-39** | |
| a189F4 | | +27 | **-15** | |
| a209A9 | AT4G12730 | **+4** | **-64** | |
| a189D6 | AT4G20260 | **+4** | **-134** | |
| a209B3 | AT2G34420 | **+5** | **-51** | |
| a197bE1 | | **+5** | **-50** | |
| a189E10 | | **+6** | **-50** | |
| a197bG5 | | **+9** | **-46** | |
| a197bA2 | | **+10** | **-45** | |
| a197bG3 | AT4G34215 | **+6** | **-110** | |
| a209B10 | AT3G26740 | **+7** | **-60** | |
| a209B2 | AT4G14450 | **+9** | irrelevant | (3) |
| a063A1 | AT3G15353 | **+9** | **-71** | |
| a192A6 | AT4G37040 | **+9** | **-27** | |
| a189E4 | AT1G17530 | **+10** | **-82** | |
| a192B9 | AT1G15820 | **+10** | **-86** | |
| a209E3 | | +25 | **-71** | |
| a189F11 | | +32 | **-64** | |
| a188H4 | AT4G00430 | +13 | **-74** | |
| a209E6 | | +26 | **-61** | |
| a189F10 | AT2G33800 | +13 | **-66** | |
| a209C4 | AT2G42070 | +18 | **-84** | |
| a192A8 | AT3G14210 | +22 | **-42** | |
| a209A12 | | +32 | **-32** | |
| a209B5 | AT1G78620 | +27 | **-10** | |
| a197bF3 | AT5G54270 | +28 | **-67** | |
| a209B12 | | +31 | **-64** | |
| a197bH2 | | +32 | **-63** | |
| a18909 | AT2G19080 | +29 | **-32** | |
| a197bF1 | AT1G20340 | +31 | **-64** | |
| a209B1 | AT2G36830 | +33 | **-60** | |
| a209C9 | AT5G10450 | +33 | **-23** | |
| a197bH3 | AT1G73230 | +38 | **-86** | |
| a209C3 | AT1G48440 | +38 | **-36** | |
| a209D7 | AT4G25130 | +39 | **-27** | (4) |
| a209C1 | AT3G26520 | +41 | **-62** | |
| a209E8 | AT4G10340 | +43 | **-52** | |
| a189C11 | | +43 | **-52** | |
| a209A3 | | +43 | **-52** | |
| a209A10 | AT5G04170 | +43 | **-43** | |
| a189B12 | AT1G28200 | +47 | **-20** | |
| a197bF5 | AT2G33040 | +48 | **-86** | |
| a189D10 | AT5G65430 | +49 | **-39** | |
| a192A7 | AT1G44810 | +54 | **-25** | |
| a197bH4 | AT1G61520 | +54 | **-88** | |
| a192D6 | | +54 | **-88** | |
| a197bA1 | | +54 | **-88** | |
| a189F1 | | +56 | **-86** | |
| a192D5 | | +59 | **-83** | |
| a188A9 | | +64 | **-78** | |
| a192D8 | | +64 | **-78** | |
| a209D3 | AT1G22300 | +55 | **-45** | |
| a065aE8 | AT4G25050 | +56 | **-54** | |
| a197bG4 | AT1G23820 | +56 | **-68** | |
| a209B11 | AT4G05180 | +56 | **-18** | |
| a189D3 | AT5G03240 | +61 | **-31** | |
| a209C10 | AT1G18080 | +61 | **-63** | |
| a189E8 | | +63 | **-61** | |
| a209A5 | AT5G01020 | +69 | **-106** | |
| a065aF7 | AT2G39390 | +73 | **-42** | |
| a063D5 | AT1G72020 | +77 | **-16** | |
| a197bH6 | AT5G48020 | +84 | **-84** | |
| a209A11 | AT1G04170 | +85 | **-116** | |
| a209C11 | AT3G26650 | +87 | **-186** | |
| a197bF4 | | +87 | **-186** | |
| a209D11 | | +87 | **-186** | |
| a192A5 | | +88 | **-185** | |
| a188E8 | | +108 | **-165** | |
| a189B8 | AT5G01530 | +112 | **-35** | |
| a192C5 | | +120 | **-27** | |
| a189F7 | | +139 | **-8** | |
| a19B11 | AT3G26070 | +139 | **-92** | |
| a209A4 | AT3G16370 | +147 | **-35** | |
| a192A12 | AT4G39090 | +148 | **-9** | |
| a209E1 | AT2G20230 | +152 | **-70** | |
| a197bA3 | AT2G43750 | +152 | **-79** | |
| a209C7 | AT3G47470 | +159 | **-412** | |
| a209D9 | | +159 | **-415** | |
| a197bF2 | AT2G45440 | +209 | **-246** | |
| a065aB1 | AT1G14320 | +243 | **-114** | |
| a209C2 | AT1G60950 | +466 | **-51** | |
| a209C6 | AT5G64250 | +202 | ∼ -190 | (5) |
| a209B6 | AT5G03880 | +27 | +2 | |
| a192D11 | AT3G53750 | | +7 | |
| a209C12 | AT2G14750 | | +30 | |
| a192D12 | AT2G20420 | | +46 | |
| a192A10 | AT5G53480 & AT5G53485 | | irrelevant | (3) |
| a192C7 | AT5G16715 | | irrelevant | (3) |
| a192C12 | AT5G02870 | | > +100 | |
| a189E3 | AT1G73940 | | > +100 | |
| a189D1 | AT4G21190 | | > +100 | |
| a192C10 | AT1G56340 | | > +100 | |
| a209B7 | AT5G19540 | | > +100 | |
| a189C12 | AT2G34510 | | > +100 | |
| a209E12 | AT1 G62750 | | > +100 | |
| a188H7 | | | > +100 | |
| a192B5 | AT1G49240 | | > +100 | |
| a192D7 | AT1G55490 | | > +100 | |
| a192D9 | AT1G65930 | | > +100 | |
| a192B12 | AT3G61440 | | > +100 | |
| a192D10 | AT2G16400 | | > +100 | |
| a209E9 | AT1G07230 | | > +100 | |

| | | | | |
|---|---|---|---|---|
| - Description du tableau 2 : Les clones (référencés en 1ère colonne) et leur région correspondante sur le génome d'Arabidopsis thaliana (Locus ID en 2^{ème} colonne) sont indiqués. La colonne Genbank (troisième colonne) donne la position relative de l'extrémité 5' des fragments clonés par rapport aux séquences publiées ; pour chaque gène, un «+1» de référence a été établi à partir de l'ADNc de Genbank qui remonte le plus loin en 5' ; les distances sont mesurées en nombre de bases d'ADNc ; les valeurs négatives indiquent un fragment cloné qui s'étend au delà du +1 en direction du promoteur et réciproquement, sachant qu'un 5' identique à la séquence de référence se traduit par la valeur +1 et que la valeur -1 indique un gain d'une base vers le promoteur. La colonne ATG (quatrième colonne) donne la position relative du 5' cloné par rapport au début de l'ORF proposé par l'AGI. En gras sont indiqués les clones d'ADNc qui couvrent la totalité de l'ORF et dont l'extrémité 5' est localisée en amont ou à moins de 10 bases en aval, du +1 de référence. La colonne note (cinquième colonne) explicite certaines lignes du tableau correspondant aux clones : (1) ADNc couvrant 2 loci, le 5' est à 8 bases en amont de l'ORF AT2G35450 et à plus de 530 bases en amont de l'ORF AT2G35440. (2) ADNc couvrant 2 loci, le 5' est à 72 bases en amont de l'ORF AT2G42240 et à 661 bases en amont de l'ORF AT2G42245. (3) Prédictions AGI incompatibles avec les données expérimentales. (4) Le clone Genbank X97326 très étendu et non épissé a été exclu de l'analyse. (5) Cet ADNc comprend la quasi-totalité de la séquence codante prédite, à l'exception des premiers codons qui font partie d'un premier intron alternatif couvert par le construit. (6) Aucun ADNc n'avait été décrit auparavant pour ce locus. | | | | |

**Tableau 3**

| | | | |
|---|---|---|---|
| clones d'ADNc analysés | | 154 | |
| genes | | 103 | |
| nouveau | | 1 | |

| Position relative de l'extrémité 5' du clone d'ADNc | | | |
|---|---|---|---|
| | a) en amont du +1 | 12 | |
| | b) identique au +1 | 20 | |
| | c) à moins de 10 bases en aval du +1 | 31 | |
| | d) en amont de l'ATG | 134 | (87%) |
| Total (a+b+c) | | 51 | (33%) |

Les fragments d'ADNc portés par les 154 construits ont pu être assignés à 103 loci distincts sur le génome d'A. thaliana. A l'exception de 5 clones, les ADNc obtenus sont globalement compatibles avec les unités de transcription et les séquences codantes annotées par l'AGI. Un total de 134 ADNc globalement compatibles avec les annotations AGI comprennent aussi l'ATG d'initiation ; le taux de clones d'ADNc couvrant une ORF complète s'élève donc à ∼87% dans cette expérience. La position relative de l'extrémité 5' des ADNc a été comparée aux séquences d'ADNc décrites dans Genbank. On prendra comme site d'initiation de transcription de référence (« +1 »), la position correspondant à la séquence d'ADNc (de Genbank) remontant le plus vers le 5'. Douze clones d'ADNc correspondent à un gain de quelques bases à plusieurs dizaines de bases en direction du promoteur. En particulier, l'ADNc a189D5 présente l'incorporation de 78 bases supplémentaires en 5' par rapport aux ADNc déjà décrits pour ce gène. Vingt clones ont des extrémités 5' identiques et 31 clones ont des 5' localisés à moins de 10 bases en aval des +1 disponibles dans Genbank pour les gènes correspondant. On notera enfin que malgré l'étendue très modeste de cet inventaire de transcrits, un clone (séquence a192B10) couvrant la totalité d'un « gène orphelin » (locus AT5G44730) a été caractérisé. L'obtention de ce transcrit qui n'était pas encore représenté parmi les quelques 221 000 séquences d'ADNc disponibles ds Genbank pour A. thaliana, peut aussi être considéré comme indicateur de la bonne sensibilité de la méthode.
Ces résultats permettent de conclure que la T4 DNA ligase peut être utilisée pour catalyser la fixation d'un oligonucléotide sur l'ARN. Un adaptateur partiellement double brin à extrémité cohésive P1/P2 ou d'un type similaire à P1/P2, permet d'obtenir un couplage efficace avec des fragments d'ARN de séquences inconnues. La stratégie de production d'ADNc pleine longueur basée notamment sur ce procédé d'étiquetage de l'ARN est validée de façon convergente par RACE PCR et par l'analyse d'une banque d'ADNc. La qualité générale des clones obtenus par les deux approches expérimentales témoigne de la bonne sensibilité de cette méthode pour caractériser l'extrémité 5' des transcrits et pour la production d'ADNc pleine longueur. En ce qui concerne la spécificité de la méthode vis-à-vis du site de rajout de la coiffe, les fragments d'ADNc « plus courts » qui ont été obtenus dans les deux types d'expérience pourraient correspondre à des sites alternatifs d'initiation de transcription ou à une inactivation incomplète par la phosphatase alcaline d'ARN messagers partiellement dégradés.

## Revendications

1. Méthode de production d'ADNc pleine longueur comprenant une étape a) de déphosphorylation des fragments d'acides nucléiques non pourvus d'une coiffe en 5', une étape b) de suppression de la coiffe de l'extrémité 5' des ARN coiffés, une étape c) de ligaturation d'oligonucléotide à l'extrémité 5' d'ARN obtenus à l'étape b) et une étape d) de synthèse des ADNc pleine longueur, par rétro-transcription des ARN obtenus à l'étape c), par une transcriptase inverse, ladite méthode étant **caractérisée en ce que** l'étape c) de ligaturation d'oligonucléotide est réalisée par une ligase et un adaptateur double brin à extrémité cohésive, ledit adaptateur double brin à extrémité cohésive comprenant un brin supérieur portant un site 3'OH accepteur de ligase pour le couplage avec le 5' des ARN et un brin inférieur présentant en 5' une extension oligonucléotidique monocaténaire de séquence aléatoire pouvant s'apparier avec l'extrémité 5' des ARN.

2. Méthode selon la revendication 1 **caractérisée en ce que** les ARN coiffés sont des ARN messagers.

3. Méthode selon la revendication 1 **caractérisée en ce que** la ligase utilisée dans l'étape c) est une DNA ligase, une RNA ligase ou une combinaison de RNA ligase et de DNA ligase.

4. Méthode selon la revendication 3 **caractérisée en ce que** la ligase utilisée est une DNA ligase.

5. Méthode selon les revendications 3 et 4 **caractérisée en ce que** la ligase utilisée est la T4 DNA ligase.

6. Méthode selon la revendication 1 **caractérisée en ce que** le brin inférieur de l'adaptateur double brin à extrémité cohésive peut être bloqué par un groupement amine en 3'.

7. Méthode selon la revendication 1 **caractérisée en ce que** les brins supérieur et inférieur de l'adaptateur double brin à extrémité cohésive peuvent être constitués d'ADN ou d'ARN ou être de composition mixte ADN/ARN.

8. Méthode selon la revendication 1 **caractérisée en ce que** le brin supérieur de l'adaptateur double brin est composé de 24 désoxyribonucléotides en 5' suivi d'un fragment de 8 ribonucléotides en 3'.

9. Méthode selon la revendication 1 **caractérisée en ce que** le brin inférieur de l'adaptateur double brin comporte en 5' une extension oligonucléotidique monocaténaire de séquence aléatoire de 6 bases.

10. Méthode de ligaturation d'un adaptateur oligonucléotidique à l'extrémité 5' d'un ARN, comprenant les étapes :
a) Obtenir un ARN simple brin présentant une extrémité 5'phosphate
b) Préparer les deux brins d'un adaptateur à extrémité cohésive comprenant un brin supérieur portant un site 3' OH accepteur de ligase pour le couplage avec le 5' des ARN et un brin inférieur à extrémité cohésive présentant en 5' une extension oligonucléotidique monocaténaire de séquence aléatoire pouvant s'apparier avec l'extrémité 5' phosphate des ARN
c) Mélanger ledit brin supérieur avec ledit brin inférieur de façon à former un adaptateur double brin
d) Mélanger ledit adaptateur avec ledit ARN simple brin pour permettre à l'extrémité 5' dudit brin inférieur de s'hybrider avec ledit simple brin d'ARN
e) Ligaturer ledit adaptateur avec ledit simple brin d'ARN par une ligase.

11. Méthode selon la revendication 10 **caractérisée en ce que** l'ARN simple brin est un ARN coiffé.

12. Méthode selon la revendication 11 **caractérisée en ce que** l'ARN coiffé est un ARN messager.

13. Méthode selon la revendication 10 **caractérisée en ce que** l'ARN simple brin est un ARN non coiffé.

14. Méthode selon la revendication 10 **caractérisée en ce que** la ligase utilisée dans l'étape e) est une DNA ligase, une RNA ligase, ou une combinaison de RNA ligase et de DNA ligase.

15. Méthode selon les revendications 10 **caractérisée en ce que** la ligase utilisée est une DNA ligase.

16. Méthode selon les revendications 10 **caractérisée en ce que** la ligase utilisée est la T4 DNA ligase.

17. Méthode selon la revendication 10 **caractérisée en ce que** le brin inférieur de l'adaptateur double brin à extrémité cohésive peut être bloqué par un groupement amine en 3'.

18. Méthode selon la revendication 10 **caractérisée en ce que** les brins supérieur et inférieur de l'adaptateur double brin à extrémité cohésive peuvent être constitués d'ADN ou d'ARN ou être de composition mixte ADN/ARN.

19. Méthode selon la revendication 10 **caractérisée en ce que** le brin supérieur de l'adaptateur double brin à extrémité cohésive est composé de 24 désoxyribonucléotides en 5' suivi d'un fragment de 8 ribonucléotides en 3.

20. Méthode selon la revendication 10 **caractérisée en ce que** le brin inférieur de l'adaptateur double brin à extrémité cohésive comporte en 5' une extension oligonucléotidique monocaténaire de séquence aléatoire de 6 bases.

21. Kit pour la production d'ADNc pleine longueur **caractérisé en ce qu'**il comprend au moins :
- phosphatase alcaline et son tampon réactionnel
- pyrophosphatase acide du tabac et son tampon réactionnel
- une ligase, ou un cocktail de ligases, comprenant en particulier la T4 DNA ligase et son tampon réactionnel
- un adaptateur double brin à extrémité cohésive, ledit adaptateur double brin à extrémité cohésive comprenant un brin supérieur portant un site 3'OH accepteur de ligase pour le couplage avec le 5' des ARN et un brin inférieur présentant en 5' une extension oligonucléotidique monocaténaire de séquence aléatoire pouvant s'apparier avec l'extrémité 5' des ARN
- une transcriptase inverse
- une ADN polymérase ADN dépendante
- des amorces oligonucléotidiques pour la polymérisation de l'ADN

## Claims

1. A method for producing full length cDNA comprising: step a), dephosphorylation of nucleic acid fragments not containing a 5'cap; step b), removal of the 5' cap from capped RNA; step c) ligation of oligonucleotide at the 5' end of the RNAs obtained in step b); and step d), synthesis of the full length cDNAs by reverse transcription of the RNAs obtained in step c) by a reverse transcriptase, said method being **characterised in that** the oligonucleotide ligation of step c) is carried out by a ligase and a double-stranded adaptor having cohesive end, said double-stranded adaptor with cohesive end comprising an upper strand carrying a 3' OH ligase acceptor site for coupling with the 5' end of RNAs and a lower strand having at the 5' end a random-sequence single-stranded oligonucleotide extension capable of pairing itself with the 5' end of RNAs.

2. The method according to claim 1, **characterised in that** the capped RNAs are messenger RNAs.

3. The method according to claim 1, **characterised in that** the ligase used in step c) is a DNA ligase, a RNA ligase or a combination of RNA ligase and DNA ligase.

4. The method according to claim 3, **characterised in that** the ligase used is a DNA ligase.

5. The method according to claims 3 and 4, **characterised in that** the ligase used is the T4 DNA ligase.

6. The method according to claim 1, **characterised in that** the lower strand of the double stranded adaptor with cohesive end can be blocked in 3' end by an amino group.

7. The method according to claim 1, **characterised in that** the upper and lower strands of the double-stranded adaptor with cohesive end can consist of DNA or RNA or mixed composition of DNA and RNA.

8. The method according to claim 1, **characterised in that** the upper strand of the double-stranded adaptor is composed of 24 deoxyribonucleotides at the 5' end followed by an 8 ribonucleotides fragment at the 3' end.

9. The method according to claim 1, **characterised in that** the lower strand of the double-stranded adaptor comprises a 6-base random-sequence single-stranded oligonucleotide extension at the 5' end.

10. A method of ligating an oligonucleotide adaptor at the 5' end of an RNA, comprising the steps:
a) Obtaining a single-stranded RNA with a phosphate 5' end
b) Preparing the two strands of an adaptor with cohesive end comprising an upper strand carring a 3' OH ligase acceptor site for the coupling with the 5' end of the RNAs and a lower strand with cohesive end, having a 5' random-sequence single-stranded oligonucleotide extension capable of pairing itself with the phosphate 5' end of RNAs
c) Mixing said upper strand with said lower strand in order to form a double-stranded adaptor
d) Mixing said adaptor with said single-stranded RNA to allow the 5' end of said lower strand to hybridise with said single-stranded RNA
e) Ligating said adaptor with said single-stranded RNA with a ligase.

11. The method according to claim 10, **characterised in that** the single-stranded RNA is a capped RNA.

12. The method according to claim 11, **characterised in that** the capped RNA is a messenger RNA.

13. The method according to claim 10, **characterised in that** the single-stranded RNA is an uncapped RNA.

14. The method according to claim 10, **characterised in that** the ligase used in step e) is a DNA ligase, a RNA ligase, or a combination of RNA ligase and DNA ligase.

15. The method according to claim 10, **characterised in that** the ligase used is a DNA ligase.

16. The method according to claim 10, **characterised in that** the ligase used is the T4 DNA ligase.

17. The method according to claim 10, **characterised in that** the lower strand of the double strand adaptor with cohesive end can be blocked in 3' end by an amino group.

18. The method according to claim 10, **characterised in that** the upper and lower strands of the double-stranded adaptor with cohesive end can consist of DNA or RNA or mixed composition of DNA and RNA.

19. The method according to claim 10, **characterised in that** the upper strand of the double-stranded adaptor with cohesive end is composed of 24 deoxyribonucleotides at the 5' end followed by a fragment of 8 ribonucleotides at the 3' end.

20. The method according to claim 10, **characterised in that** the lower strand of the double-stranded adaptor with cohesive end comprises a 6-base random-sequence single-stranded oligonucleotide extension at the 5' end.

21. A kit for producing full length cDNA **characterised in that** it comprises at least:
- alkaline phosphatase and its reaction buffer
- tobacco acid pyrophosphatase and its reaction buffer
- a ligase, or a mix of ligases, comprising in particular the T4 DNA ligase and its reaction buffer
- a double-stranded adaptor with cohesive end, said double-stranded adaptor with cohesive end comprising an upper strand carrying 3' OH ligase acceptor site for the coupling with the 5' end of RNA and a lower strand having a 5' random-sequence single-stranded oligonucleotide extension capable of pairing itself with the 5' end of RNAs
- a reverse transcriptase
- a DNA dependent DNA polymerase
- oligonucleotide primers for DNA polymerization

## Patentansprüche

1. Herstellungsmethode für vollständige cDNA, die einen Schritt a) der Dephosphorylierung der bei 5' unbedeckten Nukleinsäurefragmente, einen Schritt b) der Streichung der Bedeckung am Ende 5' der bedeckten RNA, einen Schritt c) der Oligonukleotidligation am Ende 5' der in Schritt b) hergestellten RNA und einen Schritt d) der Synthese der vollständigen cDNA durch Retrotranskription der in Schritt c) hergestellten RNA durch eine inverse Transkriptase umfasst, wobei die Methode **dadurch gekennzeichnet ist, dass** der Schritt c) der Oligonukleotidligation durch eine Ligase und einen Doppelstrang-Adapter mit kohäsivem Ende durchgeführt wird, wobei der Doppelstrang-Adapter mit kohäsivem Ende einen oberen Strang mit einer Ligase akzeptierenden 3'OH-Stelle für die Kopplung mit dem 5' der RNA und einen unteren Strang umfasst, der bei 5' eine einsträngige Oligonukleotidextension mit Zufälliger Sequenz aufweist, die sich mit dem Ende 5' der RNA paaren kann.

2. Methode nach Anspruch 1, **dadurch gekennzeichnet, dass** die bedeckten RNA Messenger-RNA sind.

3. Methode nach Anspruch 1, **dadurch gekennzeichnet, dass** die in Schritt c) verwendete Ligase eine DNA-Ligase, eine RNA-Ligase oder eine Kombination aus RNA-Ligase und DNA-Ligase ist.

4. Methode nach Anspruch 3, **dadurch gekennzeichnet, dass** die verwendete Ligase eine DNA-Ligase ist.

5. Methode nach den Ansprüchen 3 und 4, **dadurch gekennzeichnet, dass** die verwendete Ligase die T4-DNA-Ligase ist.

6. Methode nach Anspruch 1, **dadurch gekennzeichnet, dass** der untere Strang des Doppelstrangadapters mit kohäsivem Ende von einer Amingruppe bei 3' blockierbar ist.

7. Methode nach Anspruch 1, **dadurch gekennzeichnet, dass** die oberen und unteren Stränge des Doppelstrangadapters mit kohäsivem Ende aus DNA oder RNA oder aus einer gemischten DNA/RNA-Zusammensetzung bestehen können.

8. Methode nach Anspruch 1, **dadurch gekennzeichnet, dass** der obere Strang des Doppelstrangadapters aus 24 Desoxyribonukleotiden bei 5', gefolgt von einem Fragment aus 8 Ribonukleotiden bei 3', zusammengesetzt ist.

9. Methode nach Anspruch 1, **dadurch gekennzeichnet, dass** der untere Strang des Doppelstrangadapters bei 5' eine einsträngige Oligonukleotidextension mit zufälliger Sequenz von 6 Basen aufweist.

10. Ligationsmethode eines Oligonukleotidadapters am Ende 5' einer RNA, die die Schritte umfasst:
a) Herstellen einer einsträngigen RNA mit einem 5'-Phosphatende,
b) Vorbereiten der zwei Stränge eines Adapters mit kohäsivem Ende, der einen oberen Strang mit einer Ligase akzeptierenden 3'OH-Stelle für die Kopplung mit dem 5' der RNA und einen unteren Strang mit kohäsivem Ende umfasst, der bei 5' eine einsträngige Oligonukleotidextension mit zufälliger Sequenz aufweist, die sich mit dem 5'-Phospatende der RNA paaren kann,
c) Mischen des oberen Strangs mit dem unteren Strang derart, dass ein Doppelstrangadapter entsteht,
d) Mischen des Adapters mit der einsträngigen RNA, um dem Ende 5' des unteren Strangs zu erlauben, mit der einsträngigen RNA zu hybridisieren,
e) Ligation des Adapters mit der einsträngigen RNA durch eine Ligase.

11. Methode nach Anspruch 10, **dadurch gekennzeichnet, dass** die einsträngige RNA eine bedeckte RNA ist.

12. Methode nach Anspruch 11, **dadurch gekennzeichnet, dass** die bedeckte RNA eine Messenger-RNA ist.

13. Methode nach Anspruch 10, **dadurch gekennzeichnet, dass** die einsträngige RNA eine unbedeckte RNA ist.

14. Methode nach Anspruch 10, **dadurch gekennzeichnet, dass** die in Schritt e) verwendete Ligase eine DNA-Ligase, eine RNA-Ligase oder eine Kombination aus RNA-Ligase und DNA-Ligase ist.

15. Methode nach Anspruch 10, **dadurch gekennzeichnet, dass** die verwendete Ligase eine DNA-Ligase ist.

16. Methode nach Anspruch 10, **dadurch gekennzeichnet, dass** die verwendete Ligase die T4-DNA-Ligase ist.

17. Methode nach Anspruch 10, **dadurch gekennzeichnet, dass** der untere Strang des Doppelstrangadapters mit kohäsivem Ende von einer Amingruppe bei 3' blockierbar ist.

18. Methode nach Anspruch 10, **dadurch gekennzeichnet, dass** der obere und untere Strang des Doppelstrangadapters mit kohäsivem Ende aus DNA oder RNA oder aus einer gemischter DNA/RNA-Zusammensetzung bestehen kann.

19. Methode nach Anspruch 10, **dadurch gekennzeichnet, dass** der obere Strang des Doppelstrangadapters mit kohäsivem Ende aus 24 Desoxyribonukleotiden bei 5' gefolgt von einem Fragment aus 8 Ribonukleotiden bei 3 zusammengesetzt ist.

20. Methode nach Anspruch 10, **dadurch gekennzeichnet, dass** der untere Strang des Doppelstrangadapters mit kohäsivem Ende bei 5' eine einsträngige Oligonukleotidextension mit zufälliger Sequenz von 6 Basen aufweist.

21. Kit zur Herstellung von vollständiger cDNA, **dadurch gekennzeichnet, dass** es mindestens umfasst:
- Alkalinphosphatase und ihren Reaktionspuffer,
- saure Pyrophosphatase des Tabaks und ihren Reaktionspuffer,
- eine Ligase oder eine Ligasemischung, der insbesondere die T4-DNA-Ligase und ihren Reaktionspuffer umfasst,
- einen Doppelstrangadapter mit kohäsivem Ende, wobei der Doppelstrangadapter mit kohäsivem Ende einen oberen Strang mit einer Ligase akzeptierenden 3'OH-Stelle für die Kopplung mit dem 5' der RNA und einen unteren Strang umfasst, der bei 5' eine einsträngige Oligonukleotidextension mit zufälliger Sequenz aufweist, die sich mit dem 5'-Ende der RNA paaren kann,
- eine inverse Transkriptase,
- eine DNA-abhängige DNA-Polymerase,
- Oligonukleotid-Primer für die Polymerisation der DNA.


### LISTE DE SEQUENCES

<110> CENTRE NATIONAL DE LA RECHERCHE SCIENTIFIQUE (CNRS)
<120> Méthode de production d'ADNc pleine longueur par ligaturation d'adaptateur à extrémité cohésive
<130> D21714
<160> 11
<170> PatentIn version 3.2
<210> 1
   <211> 32
   <212> ADN
   <213> Séquence artificielle
<220>
   <221> source
   <222> (1)..(32)
   <223> /note= "Description de la séquence artificielle: brin supérieur d'un adaptateur double brin à extrémité cohésive (P1)"
<220>
   <221> misc_feature
   <222> (25)..(32)
   <223> ribonucléotides
<400> 1
   ggggacaagt ttgtacaaaa aagcaggctg gg 32
<210> 2
   <211> 16
   <212> ADN
   <213> Séquence artificielle
<220>
   <221> source
   <222> (1)..(16)
   <223> /note= "Description de la séquence artificielle: brin inférieur d'un adaptateur double brin à extrémité cohésive (P2)"
<220>
   <221> misc feature
   <222> (1)..(6)
   <223> n correspond à n'importe quel type de base pouvant s'apparier avec l'ARN.
<220>
   <221> misc_feature
   <222> (16)..(16)
   <223> Groupement amine sur le carbone 3'
<400> 2
   nnnnnnccca gcctgc 16
<210> 3
   <211> 50
   <212> ADN
   <213> Séquence artificielle
<220>
   <221> source
   <222> (1)..(50)
   <223> /note= "Description de la séquence artificielle: amorce de rétro transcription P3"
<220>
   <221> misc_feature
   <222> (50)..(50)
   <223> n correspond à n'importe quel type de base pouvant s'apparier avec l'ARN
<400> 3
   ggggaccact ttgtacaaga aagctgggtt tttttttttt ttttttttvn 50
<210> 4
   <211> 21
   <212> ADN
   <213> Séquence artificielle
<220>
   <221> source
   <222> (1)..(21)
   <223> /note= "Description de la séquence artificielle: amorce de PCR Rbc64.U"
<400> 4
   aatggcttcc tctatgctct c 21
<210> 5
   <211> 24
   <212> ADN
   <213> Séquence artificielle
<220>
   <221> source
   <222> (1)..(24)
   <223> /note= "Description de la séquence artificielle: amorce de PCR Rbc402.L"
<400> 5
   tgcggagaag gtagtcaact tcct 24
<210> 6
   <211> 20
   <212> ADN
   <213> Séquence artificielle
<220>
   <221> source
   <222> (1)..(20)
   <223> /note= "Description de la séquence artificielle: amorce de PCR M13fwd"
<400> 6
   tgtaaaacga cggccagtga 20
<210> 7
   <211> 21
   <212> ADN
   <213> Séquence artificielle
<220>
   <221> source
   <222> (1)..(21)
   <223> /note= "Description de la séquence artificielle: amorce de PCR M13rev"
<400> 7
   ggaaacagct atgaccatga t 21
<210> 8
   <211> 22
   <212> ADN
   <213> Séquence artificielle
<220>
   <221> source
   <222> (1)..(22)
   <223> /note= "Description de la séquence artificielle: amorce de PCR Univ221.U"
<400> 8
   tgtaaaacga cggccagtct ta 22
<210> 9
   <211> 22
   <212> ADN
   <213> Séquence artificielle
<220>
   <221> source
   <222> (1)..(22)
   <223> /note= "Description de la séquence artificielle: amorce de PCR Univ221.L"
<400> 9
   caggaaacag ctatgaccat gt 22
<210> 10
   <211> 29
   <212> ADN
   <213> Séquence artificielle
220>
   <221> source
   <222> (1)..(29)
   <223> /note= "Description de la séquence artificielle: amorce de PCR Piper"
<400> 10
   ggggacaagt ttgtacaaaa aagcaggct 29
<210> 11
   <211> 29
   <212> ADN
   <213> Séquence artificielle
<220>
   <221> source
   <222> (1)..(29)
   <223> /note= "Description de la séquence artificielle: amorce de PCR P3pcr"
<400> 11
   ggggaccact ttgtacaaga aagctgggt 29